# EUROPEAN PATENT APPLICATION

(11) **EP 1 975 826 A2**
(43) Date of publication of application: **01.10.2008**
(21) Application number: 08000170.4
(22) Date of filing: 07.01.2008
(51) Int. Cl.: G06F 19/00

(54) **System controller**

(30) Priority: 29.03.2007 JP 2007089015
(71) Applicant: OLYMPUS MEDICAL SYSTEMS CORP., Shibuya-ku Tokyo 151-0072 (JP)
(72) Inventor: Hibi, Yasushi, Tokyo 151-0072 (JP)
(74) Representative: Thum, Bernhard

(57) **Abstract**

A system controller according to the present invention communicates with a medical control device connectable to plural types of ultrasonic endoscopes. The system controller includes a plurality of operation instruction units, a communication unit, and a control unit. The plurality of operation instruction units are capable of issuing an instruction to the medical control device. The communication unit is capable of receiving a control command generated by the medical control device on the basis of table data representing the correspondence among the connection state of the ultrasonic endoscopes, the operational state of the medical control device, and the state of each of the plurality of operation instruction units. On the basis of the control command, the control unit performs a control to visually reflect, in each of the plurality of operation instruction units, the use state of respective functions achievable by the ultrasonic endoscopes and the medical control device.

## Description

### BACKGROUND OF THE INVENTION

### 1. Field of the Invention

The present invention relates to a system controller for communicating with a medical control device connected to an ultrasonic endoscope.

### 2. Description of the Related Art

Conventionally, an ultrasonic diagnostic apparatus has been widely used which transmits ultrasonic waves into a living body as a subject to be examined and receives reflected waves generated by reflection of the ultrasonic waves in a living tissue constituting a site to be examined in the living body to thereby obtain a tomographic image of the living body. The tomographic image of the living body obtained by the ultrasonic diagnostic apparatus is used in, for example, diagnosis of the invasion depth of a lesion, observation of the internal state of an organ, or the like performed by a user, such as a surgeon.

As an example of the above-described apparatus for obtaining a tomographic image of a living body, an ultrasonic diagnostic apparatus system proposed in Japanese Unexamined Patent Application Publication No. 2005-177348 is widely known.

Further, in ultrasonic diagnostic apparatuses of recent years, along with the diversification of the functions thereof, keys, switches, and so forth included in a user interface device such as a keyboard, for example, which is a device capable of issuing a variety of instructions to use desired functions, have been increasing in number.

However, due to a large number of keys, switches, and so forth included in a keyboard disclosed in Japanese Unexamined Patent Application Publication No. 2005-177348, it is difficult to visually determine at a glance whether or not the respective functions of the ultrasonic diagnostic apparatus system are usable. As a result, there is a problem of reduced operability.

The present invention has been made in view of the above-described circumstances, and an object of the present invention is to provide a system controller capable of improving the operability with respect to a medical control device more than before.

### SUMMARY OF THE INVENTION

A system controller according to an aspect of the present invention is a system controller for communicating with a medical control device connectable to plural types of ultrasonic endoscopes. The system controller includes a plurality of operation instruction units, a communication unit, and a control unit. The plurality of operation instruction units are capable of issuing an instruction to the medical control device. The communication unit is capable of receiving a control command generated by the medical control device on the basis of table data representing the correspondence among the connection state of the ultrasonic endoscopes, the operational state of the medical control device, and the state of each of the plurality of operation instruction units. On the basis of the control command, the control unit performs a control to visually reflect, in each of the plurality of operation instruction units, the use state of respective functions achievable by the ultrasonic endoscopes and the medical control device.

### BRIEF DESCRIPTION OF THE DRAWINGS

Fig. 1 is a diagram illustrating an example of a configuration of main parts of a medial system using a system controller according to an embodiment of the present invention;
Fig. 2 is a diagram illustrating an example of an external view of the system controller according to the embodiment of the present invention;
Fig. 3 is a diagram illustrating an example of table data representing the correspondence among the connection state of ultrasonic endoscopes, the operational state of a processor, and the state of respective operation instruction units included in the system controller;
Fig. 4 is a diagram illustrating an example of table data representing the correspondence between a control command outputted from the processor and the state of the respective operation instruction units included in the system controller;
Fig. 5 is a diagram illustrating an example of a screen pattern displayed on a touch panel included in the system controller according to the embodiment of the present invention;
Fig. 6 is a diagram illustrating a different example from the example of Fig. 5 of the screen pattern displayed on the touch panel included in the system controller according to the embodiment of the present invention;
Fig. 7 is a diagram illustrating a different example from the examples of Figs. 5 and 6 of the screen pattern displayed on the touch panel included in the system controller according to the embodiment of the present invention; and
Fig. 8 is a diagram illustrating a different example from the examples of Figs. 5, 6, and 7 of the screen pattern displayed on the touch panel included in the system controller according to the embodiment of the present invention.

### DETAILED DESCRIPTION OF THE PREFERRED EMBODIMENT

An embodiment of the present invention will be described below with reference to the drawings.

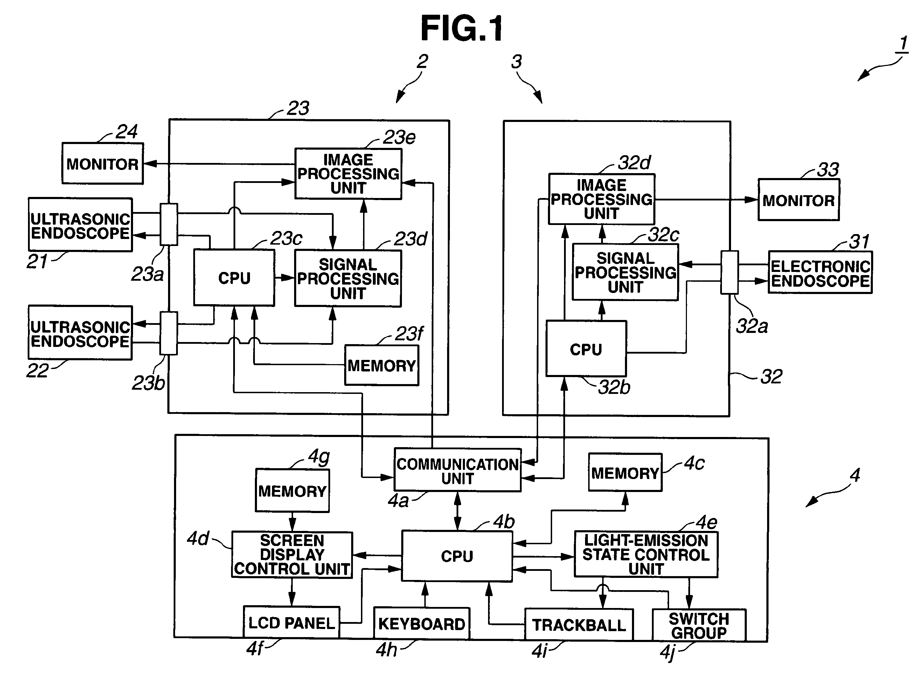

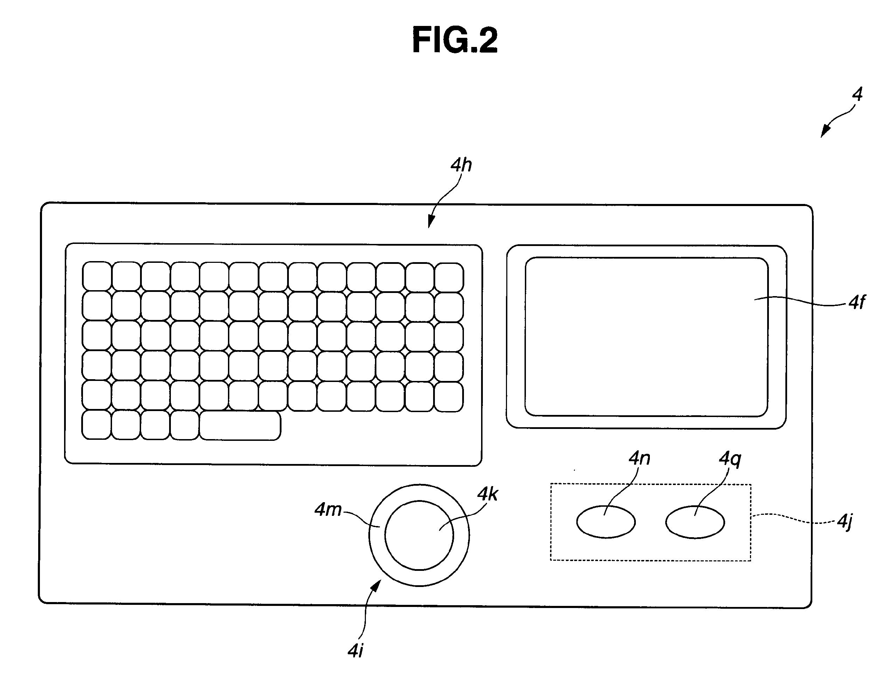

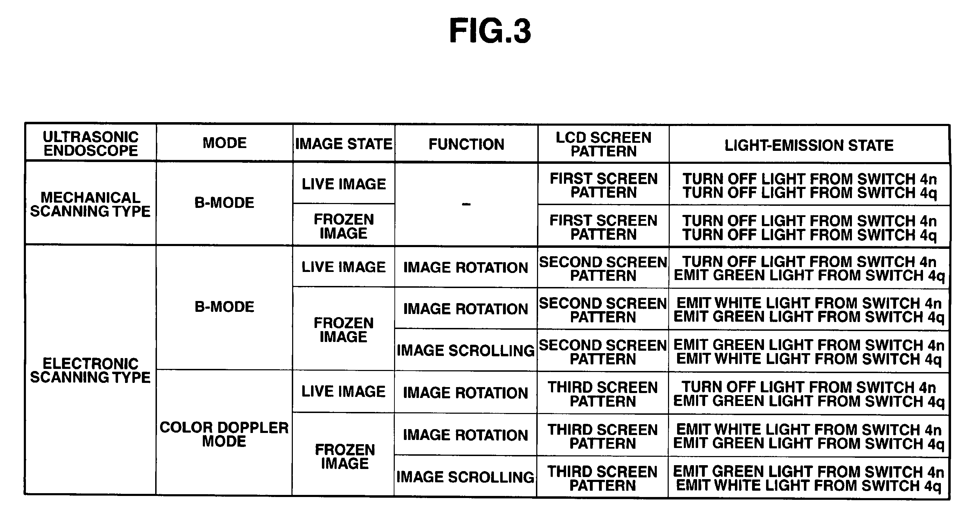

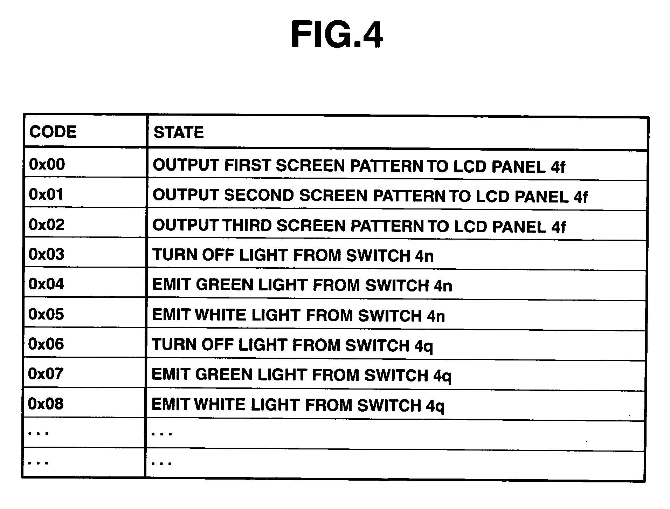

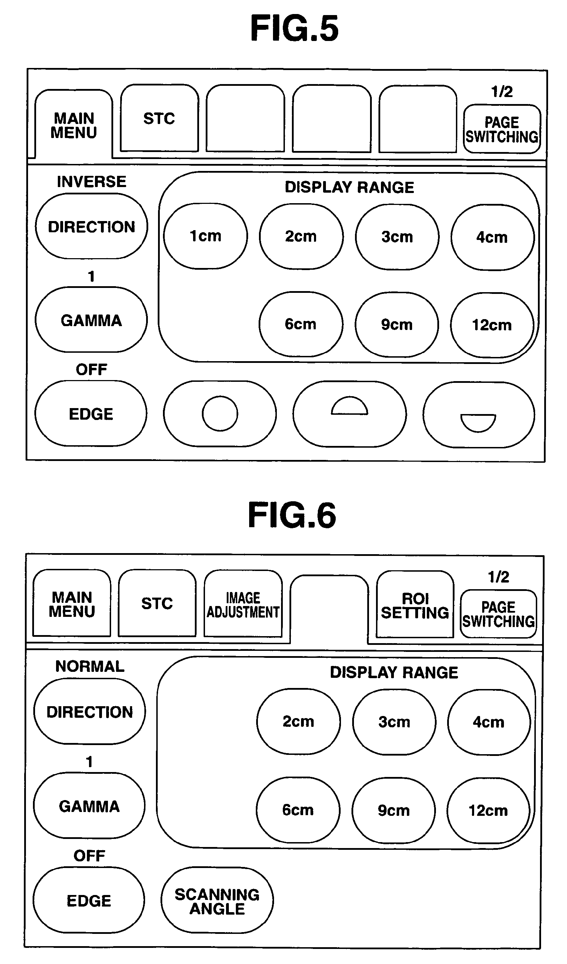

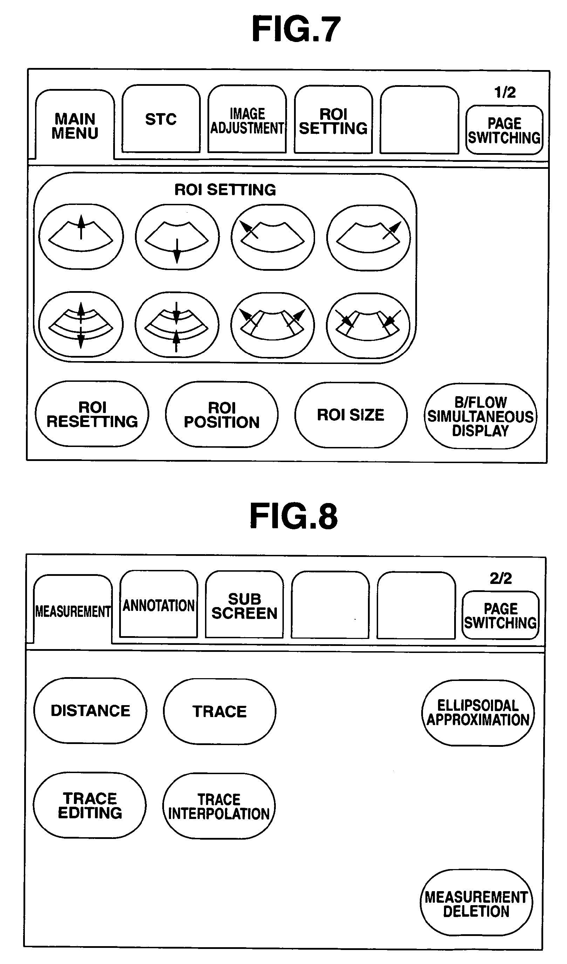

Figs. 1 to 8 relate to the embodiment of the present invention. Fig. 1 is a diagram illustrating an example of a configuration of main parts of a medial system using a system controller according to the embodiment of the present invention. Fig. 2 is a diagram illustrating an example of an external view of the system controller according to the embodiment of the present invention. Fig. 3 is a diagram illustrating an example of table data representing the correspondence among the connection state of ultrasonic endoscopes, the operational state of a processor, and the state of respective operation instruction units included in the system controller. Fig. 4 is a diagram illustrating an example of table data representing the correspondence between a control command outputted from the processor and the state of the respective operation instruction units included in the system controller. Fig. 5 is a diagram illustrating an example of a screen pattern displayed on a touch panel included in the system controller according to the embodiment of the present invention. Fig. 6 is a diagram illustrating a different example from the example of Fig. 5 of the screen pattern displayed on the touch panel included in the system controller according to the embodiment of the present invention. Fig. 7 is a diagram illustrating a different example from the examples of Figs. 5 and 6 of the screen pattern displayed on the touch panel included in the system controller according to the embodiment of the present invention. Fig. 8 is a diagram illustrating a different example from the examples of Figs. 5, 6, and 7 of the screen pattern displayed on the touch panel included in the system controller according to the embodiment of the present invention.

As illustrated in Fig. 1, a medical system 1 is configured to include an ultrasonic endoscope system 2, an electronic endoscope system 3, and a system controller 4. The ultrasonic endoscope system 2 is capable of obtaining a tomographic image of an internal part of a living body as a subject to be examined. The electronic endoscope system 3 is capable of obtaining an optical image of the internal part of the living body. The system controller 4 is capable of issuing an instruction to and communicating with the ultrasonic endoscope system 2 and the electronic endoscope system 3.

As illustrated in Fig. 1, the ultrasonic endoscope system 2 includes, as main parts thereof, an ultrasonic endoscope 21, an ultrasonic endoscope 22, a processor 23, and a monitor 24. The ultrasonic endoscope 21 is provided with a mechanical scanning type ultrasonic transducer at a distal end portion of an insertion section thereof which can be inserted into the living body. The ultrasonic endoscope 22 is provided with an electronic scanning type ultrasonic transducer at a distal end portion of an insertion section thereof which can be inserted into the living body.
The processor 23 is connected to the ultrasonic endoscopes 21 and 22 via not-illustrated cables, and generates a tomographic image in accordance with an echo signal outputted from the ultrasonic endoscopes 21 and 22. The monitor 24 displays the tomographic image.

The processor 23 having functions of a medical control device includes a connector 23a, a connector 23b, a CPU (Central Processing Unit) 23c, a signal processing unit 23d, an image processing unit 23e, and a memory 23f. The connector 23a is capable of connecting the ultrasonic endoscope 21 to the processor 23. The connector 23b is capable of connecting the ultrasonic endoscope 22 to the processor 23. The signal processing unit 23d performs such processes as wave detection and gain adjustment on the echo signal outputted from the ultrasonic endoscopes 21 and 22. The image processing unit 23e generates a tomographic image in accordance with an echo signal outputted from the signal processing unit 23d, and outputs the tomographic image as a moving image or a still image in accordance with the control by the CPU 23c. The memory 23f stores first table data representing the correspondence between respective functions usable in the ultrasonic endoscopes 21 and 22 and the state of the system controller 4.

The CPU 23c detects whether or not the ultrasonic endoscope 21 is connected to the connector 23a, whether or not the ultrasonic endoscope 22 is connected to the connector 23b, and the operational state of the processor 23. Then, on the basis of the result of the detection, the CPU 23c outputs to the system controller 4 a control command in accordance with the first table data stored in the memory 23f. Further, upon detection of the input of an instruction signal, a key code, or the like for changing the function or the mode to be used, the CPU 23c reads the first table data stored in the memory 23f, and outputs to the system controller 4 a control command in accordance with the instruction signal, the key code, or the like.

Further, in accordance with an instruction signal, a key code, and so forth outputted from the system controller 4, the CPU 23c performs controls on respective parts included in the ultrasonic endoscopes 21 and 22 and the processor 23.

Furthermore, on the basis of an instruction signal outputted from the system controller 4, for example, the CPU 23c performs a control on the image processing unit 23e to switch the tomographic image outputted to the monitor 24 to one of the moving image and the still image.

As illustrated in Fig. 1, the electronic endoscope system 3 includes, as main parts thereof, an electronic endoscope 31, a processor 32, and a monitor 33. The electronic endoscope 31 is provided with an objective optical system and an image pickup device at a distal end portion of an insertion section thereof which can be inserted into the living body. The processor 32 generates an optical image in accordance with an image pickup signal outputted from the electronic endoscope 31. The monitor 33 displays the optical image.

The processor 32 includes a connector 32a, a CPU 32b, a signal processing unit 32c, and an image processing unit 32d. The connector 32a is capable of connecting the electronic endoscope 31 to the processor 32. The signal processing unit 32c performs such processes as noise removal on the image pickup signal outputted from the electronic endoscope 31. The image processing unit 32d generates and outputs an optical image in accordance with an image pickup signal outputted from the signal processing unit 32c.

The CPU 32b performs controls on respective parts included in the electronic endoscope 31 and the processor 32 in accordance with an instruction signal, a key code, and so forth outputted from the system controller 4.

The system controller 4 can be connected to the processors 23 and 32 via not-illustrated cables. Further, as illustrated in Fig. 1, the system controller 4 includes a communication unit 4a, a CPU 4b, a memory 4c, a screen display control unit 4d, a light-emission state controlling unit 4e, an LCD (Liquid Crystal Display) panel 4f, a memory 4g, a keyboard 4h, a trackball 4i, and a switch group 4j. The memory 4c stores second table data representing the correspondence between the control command outputted from the processor 23 and the state of the system controller 4. The memory 4g stores a variety of image data used to constitute a display screen of the LCD panel 4f.

As illustrated in Fig. 2, the trackball 4i includes a ball member 4k and a ring-shaped light-emitting portion 4m disposed around an outer circumferential portion of the ball member 4k.

As illustrated in Fig. 2, the switch group 4j, which serves as a switch unit constituting operation instruction units, includes switches 4n and 4q, each of which can emit or turn off light in accordance with the control by the light-emission state control unit 4e and output an instruction signal in accordance with an allocated function. Further, each of the switches 4n and 4q, which is configured to be able to emit light in a plurality of different colors, includes therein a plurality of LEDs (Light-Emitting Diodes) having the function of a lighting portion, for example.

It is assumed in the present embodiment that the switch 4n is configured as a mechanical switch capable of outputting an instruction signal for turning on or off an image scrolling function for scrolling an image displayed on the monitor 24 in the vertical and horizontal directions, and that the switch 4q is configured as a mechanical switch capable of outputting an instruction signal for turning on or off an image rotating function for rotating the image displayed on the monitor 24. It is also assumed in the present embodiment that the image scrolling function and the image rotating function described above cannot be simultaneously used for a single image displayed on the monitor 24.

Each of the switches included in the switch group 4j is not limited to a switch allocated with only one function corresponding to the ultrasonic endoscope system 2. For example, therefore, each of the switches may be configured as a switch further allocated with another function corresponding to the electronic endoscope system 3, in addition to the above function. Specifically, each of the switches included in the switch group 4j may be configured to be able to output an instruction signal representing one function corresponding to the ultrasonic endoscope system 2 in a normal press of the switch, and to be able to output an instruction signal representing another function corresponding to the electronic endoscope system 3 in a press-and-hold of the switch.

As a process for communication of the system controller 4 with both processors 23 and 32, the communication unit 4a performs such processes as a protocol conversion process, for example, on each of signals inputted thereto, and outputs resultant signals. The above-described protocol conversion process is performed in accordance with the result of detection of the state of a not-illustrated dip switch included in the system controller 4 or the result of detection of the state of the power supplies of the processors 23 and 32 connected to the system controller 4. Accordingly, even if the two processors 23 and 32 use mutually different protocols, the system controller 4 can appropriately mediate communication between the two processors.

With the communication unit 4a having the above-described configuration, the medical system 1 according to the present embodiment can perform, for example, communication in which a video signal generated in the processor included in one of the endoscope systems is outputted via the system controller 4 to the processor and the monitor included in the other endoscope system. Further, due to the above-described communication available in the medical system 1 according to the present embodiment, it is possible in the medical system 1 to display the tomographic image generated by the processor 23 and the optical image generated by the processor 32 on the monitor 24 as a PinP (Picture-in-Picture) image, and to display the tomographic image and the optical image on the monitor 24 by switching between the images.

Processes and so forth relating to the display of the PinP image may be performed by a desired processor selected by a user, or may be performed by a processor selected by the CPU 4b of the system controller 4 on the basis of the connection state of the respective processors.

Further, the communication unit 4a is configured to be able to receive a variety of control commands outputted from the processor 23 and output the control commands to the CPU 4b, and to be able to transmit to the processors 23 and 32 the key codes and so forth outputted from the CPU 4b.

The CPU 4b constituting a part of a control unit of the present embodiment scans the input state of the keyboard 4h, and outputs a key code in accordance with the input state to the CPUs 23c and 32b via the communication unit 4a. Further, the CPU 4b outputs an instruction signal in accordance with the operation of the LCD panel 4f, the trackball 4i, and the switch group 4j to the CPUs 23c and 32b via the communication unit 4a.

On the basis of the control command inputted via the communication unit 4a, the CPU 4b performs a control and so forth on the screen display control unit 4d and the light-emission state control unit 4e to have the respective interfaces included in the system controller 4 (the LCD panel 4f, the keyboard 4h, the trackball 4i, and the switch group 4j) correspond to the state of the second table data read from the memory 4c.

In the present embodiment, the CPU 4b may be configured to determine, upon detection of the input of an image switching instruction signal for switching the image displayed on the monitor 24 from the optical image to the tomographic image, for example, whether or not an ultrasonic wave emission instruction signal for causing ultrasonic waves to be emitted from an ultrasonic endoscope connected to the processor 23 should be outputted to the processor 23 together with the image switching instruction signal, on the basis of a setting content previously stored in the memory 4c.

The screen display control unit 4d constituting a part of the control unit of the present embodiment reads the image data from the memory 4g on the basis of the control by the CPU 4b, and performs a control to appropriately change a screen display state of the LCD panel 4f in accordance with the image data.

On the basis of the control by the CPU 4b, the light-emission state control unit 4e constituting a part of the control unit of the present embodiment performs a control to appropriately change the light-emission states of the respective parts of the switch group 4j and the ring-shaped light-emitting portion 4m included in the trackball 4i.

As described above, the control unit of the system controller 4 according to the present embodiment is configured to include the CPU 4b, the screen display control unit 4d, and the light-emission state control unit 4e.

The LCD panel 4f, which is configured as a touch panel, changes the screen display state in accordance with the control by the screen display control unit 4d, and outputs to the CPU 4b an instruction signal in accordance with the pressing of each of the switches displayed in the screen, which serve as the switch unit constituting the operation instruction units. The LCD panel 4f may be configured to operate in conjunction with a not-illustrated buzzer included therein so as to produce mutually different sounds for the pressing of a valid switch and the pressing of an invalid switch, for example.

As described above, the operation instruction units of the system controller 4 according to the present embodiment are configured to include at least the respective switches included in the switch group 4j and the respective switches displayed in the screen of the LCD panel 4f.

The keyboard 4h includes a general key group enabling the input of numbers and letters, and a special key group enabling operations relating to predetermined functions, such as the output of an image. If the user presses down a predetermined key included in the general key group while holding down a "VTR (Video Tape Recorder)/Printer" key included in the special key group, for example, a key code for performing an operation such as the replay, the fast-forward, and the rewind of a VTR, i.e., a key code in accordance with the combination of the "VTR/Printer" key and the predetermined key can be outputted to the CPU 4b.

The ring-shaped light-emitting portion 4m included in the trackball 4i is formed by an LED and so forth. On the basis of the control by the light-emission state control unit 4e, the ring-shaped light-emitting portion 4m changes the light-emission state thereof in accordance with whether or not a predetermined operation relating to the image displayed on the monitor 24 (e.g., an image scrolling operation or an image rotating operation) can be performed by the ball member 4k.

Subsequently, description will be made of an operation of the medical system 1 according to the present embodiment.

A user first connects one or more of the endoscopes of the medical system 1 (the ultrasonic endoscopes 21 and 22 and the electronic endoscope 31) suitable for a desired observation content to predetermined connectors (the connectors 23a, 23b, and 32a) of the respective processors (the processors 23 and 32). Thereafter, the user turns on the power supplies of the respective parts included in the medical system 1.

Upon turn-on of the power supply of the processor 23, the CPU 23c detects whether or not the ultrasonic endoscopes 21 and 22 are connected to the connectors 23a and 23b, respectively, and also detects the operational state of the processor 23. Then, on the basis of the result of the detection, the CPU 23c outputs to the system controller 4 a control command in accordance with the first table data stored in the memory 23f.

The memory 23f stores, as the first table data, table data as illustrated in Fig. 3, for example.

Specifically, the first table data is table data associating the type of ultrasonic endoscope connected to the processor 23, the type of mode usable by the ultrasonic endoscope, the type of image outputted from the processor 23, and the type of function used in the ultrasonic endoscope with the screen pattern displayed on the LCD panel 4f and the light-emission state of the switch group 4j.

For example, upon detection that the ultrasonic endoscope 21 is connected to the connector 23a, the CPU 23c determines that only the B (Brightness)-mode is usable. Then, on the basis of the result of the determination, the CPU 23c sets the screen pattern displayed on the LCD panel 4f to a first screen pattern, sets both switches 4n and 4q to turn off the light of the switches, and outputs to the system controller 4 a control command in accordance with the setting content.

Further, for example, upon detection that the ultrasonic endoscope 22 is connected to the connector 23b, the CPU 23c determines that the B-mode and the color Doppler mode are both usable. Thereafter, the CPU 23c further detects which mode of the B-mode and the color Doppler mode is used to operate the processor 23, and which one of a live image (a moving image) and a frozen image (a still image) is the image outputted from the processor 23, to thereby determine the setting of the screen pattern displayed on the LCD panel 4f and the light-emission state of the switch group 4j. Then, the CPU 23c outputs to the system controller 4 a control command in accordance with the setting content.

On the basis of the control command outputted from the CPU 23c of the processor 23 and inputted through the communication unit 4a, the CPU 4b of the system controller 4 performs a control and so forth on the screen display control unit 4d and the light-emission state control unit 4e to have the respective interfaces included in the system controller 4 (the LCD panel 4f, the keyboard 4h, the trackball 4i, and the switch group 4j) correspond to the state of the second table data read from the memory 4c.

The memory 4c stores, as the second table data, table data as illustrated in Fig. 4, for example.

Specifically, the second table data is table data associating the respective codes included in the control command outputted from the CPU 23c with the states of the respective interfaces included in the system controller 4 (the LCD panel 4f, the keyboard 4h, the trackball 4i, and the switch group 4j).

On the basis of the control by the CPU 4b, the screen display control unit 4d reads the image data from the memory 4g, and performs a control to appropriately change the screen display state of the LCD panel 4f in accordance with the image data.

The memory 4g stores, as the above-described image data, image data in accordance with respective screen patterns illustrated in Figs. 5 to 8, for example.

Fig. 5 illustrates the first screen pattern (corresponding to "FIRST SCREEN PATTERN" described in Figs. 3 and 4) displayed on the LCD panel 4f in a state in which the ultrasonic endoscope 21 is connected to the connector 23a. The first screen pattern includes, in a "MAIN MENU" tag, switches capable of changing the range of scanning by the ultrasonic endoscope 21 (e.g., a half or whole circumference), the range of display of the image outputted to the monitor 24, and so forth.

Further, in addition to the above-described "MAIN MENU" tag, the first screen pattern includes a "STC (Sensitivity Time Control)" tag including (not-illustrated) switches relating to sensitivity adjustment. By pressing down one of the tags shown in the first screen pattern displayed on the LCD panel 4f, the user can view and press down the respective switches included in the tag.

Fig. 6 illustrates the second screen pattern (corresponding to "SECOND SCREEN PATTERN" described in Figs. 3 and 4) displayed on the LCD panel 4f in a state in which the ultrasonic endoscope 22 is connected to the connector 23b and the processor 23 operates in the B-mode. The second screen pattern includes, in the "MAIN MENU" tag, switches capable of changing the angle of scanning by the ultrasonic endoscope 22, the range of display of the image outputted to the monitor 24, and so forth.

Further, in addition to the above-described "MAIN MENU" tag, the second screen pattern includes the "STC" tag including switches relating to sensitivity adjustment and an "IMAGE ADJUSTMENT" tag including switches relating to image adjustment. By pressing down one of the tags shown in the second screen pattern displayed on the LCD panel 4f, the user can view and press down the respective switches included in the tag.

In the present embodiment, a "1 cm" switch of a "DISPLAY RANGE" field included in the "MAIN MENU" tag of the first screen pattern illustrated in Fig. 5 represents a function usable only in the ultrasonic endoscope 21. In other words, the "1 cm" switch represents a function unusable in the ultrasonic endoscope 22. Thus, due to the control by the screen display control unit 4d, the "1 cm" switch is not included in the "MAIN MENU" tag of the second screen pattern illustrated in Fig. 6 (not displayed on the LCD panel 4f), and is invalidated.

In the present embodiment, when the display range of the image displayed on the monitor 24 is changed (to a larger value, for example) by the pressing of one of the switches included in the "DISPLAY RANGE" field of the first screen pattern illustrated in Fig. 5, the frequency of the ultrasonic waves emitted from the ultrasonic endoscope 21 may be automatically changed (to a smaller value, for example). Further, in the present embodiment, the "DISPLAY RANGE" field of the first screen pattern illustrated in Fig. 5 may display, for example, only switches representing ranges adjustable in accordance with the frequency of the ultrasonic waves emitted from the ultrasonic endoscope 21.

In the present embodiment, when the display range of the image displayed on the monitor 24 is changed (to a larger value, for example) by the pressing of one of the switches included in the "DISPLAY RANGE" field of the second screen pattern illustrated in Fig. 6, the frequency of the ultrasonic waves emitted from the ultrasonic endoscope 22 may be automatically changed (to a smaller value, for example). Further, in the present embodiment, the "DISPLAY RANGE" field of the second screen pattern illustrated in Fig. 6 may display, for example, only switches representing ranges adjustable in accordance with the frequency of the ultrasonic waves emitted from the ultrasonic endoscope 22.

In the present embodiment, when the frequency of the ultrasonic waves emitted from the ultrasonic endoscope 21 or 22 is changed (by the operation of a predetermined key or the like included in the system controller 4, for example), the display range of the image displayed on the monitor 24 may be automatically set to an optimal range (which includes other ranges than the ranges included as the respective switches included in the "DISPLAY RANGE" field of each of the screen patterns illustrated in Figs. 5 and 6).

Fig. 7 illustrates the third screen pattern (corresponding to "THIRD SCREEN PATTERN" described in Figs. 3 and 4) displayed on the LCD panel 4f in a state in which the ultrasonic endoscope 22 is connected to the connector 23b and the processor 23 operates in the color Doppler mode. In addition to the respective tags included in the second screen pattern illustrated in Fig. 6, the third screen pattern further includes a "ROI (Region of Interest) SETTING" tag.

The "ROI SETTING" tag includes switches capable of, for example, changing the position of a ROI, changing the size of the ROI, and switching simultaneous display of a B-mode image and a color flow image on the monitor 24.

That is, on the basis of the control by the CPU 4b, the screen display control unit 4d appropriately changes the number of tags and switches displayed on the LCD panel 4f in accordance with the number of functions achievable by the processor 23 and the ultrasonic endoscope connected to the processor 23.

Further, when the user presses down a "PAGE SWITCHING" switch included in each of the screen patterns illustrated in Figs. 5 to 7, an instruction signal in accordance with the pressing is outputted to the CPU 4b. Then, on the basis of the instruction signal, the CPU 4b performs a control on the screen display control unit 4d to cause the unit to output the fourth screen pattern illustrated in Fig. 8 to the LCD panel 4f. Thereby, the LCD panel 4f displays an image in accordance with the fourth screen pattern.

The fourth screen pattern illustrated in Fig. 8 includes tags and switches relating to functions usable in both the ultrasonic endoscopes 21 and 22 but less frequently used than the tags and switches of the respective screen patterns illustrated in Figs. 5 to 7. Specifically, in addition to the "PAGE SWITCHING" switch, the fourth screen pattern includes, for example, a "MEASUREMENT" tag including switches relating to the measurement of the distance and so forth, an "ANNOTATION" tag including switches relating to additional information, and a "SUBSCREEN" tag including switches relating to the output of an image to a monitor other than the monitor 24.

It is assumed in the present embodiment that, when the user presses down the "PAGE SWITCHING" switch included in the fourth screen pattern illustrated in Fig. 8, the CPU 4b and the screen display control unit 4d perform a control and so forth to switch the screen displayed on the LCD panel 4f back to the original screen (one of the screen patterns illustrated in Figs. 5 to 7).

The system controller 4 according to the present embodiment may be configured such that the function allocated to each of the switches included in the screen patterns illustrated in Figs. 5 to 8 can be allocated to, for example, one of the keys included in the keyboard 4h or one of the switches included in the switch group 4j.

To save power consumption, the system controller 4 according to the present embodiment may be configured, for example, such that the screen display control unit 4d performs a control to temporarily turn off the display of the LCD panel 4f when it is detected that an operation on the screen displayed on the LCD panel 4f (e.g., the pressing of a switch) has not been performed for a predetermined period, and to thereafter turn on the display of the LCD panel 4f when the operation on the screen displayed on the LCD panel 4f (e.g., the pressing of a switch) is again performed.

Meanwhile, on the basis of the control by the CPU 4b, the light-emission state control unit 4e performs a control to appropriately change the light-emission states of the respective parts of the switch group 4j and the ring-shaped light-emitting portion 4m included in the trackball 4i.

On the basis of the control by the light-emission state control unit 4e, the ring-shaped light-emitting portion 4m emits light in the ON state of one of the switches 4n and 4q (one of the image scrolling function and the image rotating function), for example, to visually indicate that an operation by the ball member 4k on the image and so forth displayed on the monitor 24 can be performed. Further, on the basis of the control by the light-emission state control unit 4e, the ring-shaped light-emitting portion 4m turns off light in the OFF state of both of the switches 4n and 4q, for example, to visually indicate that the operation by the ball member 4k on the image and so forth displayed on the monitor 24 cannot be performed.

On the basis of the control by the light-emission state control unit 4e, the switch 4n included in the switch group 4j emits light in green color (corresponding to "EMIT GREEN LIGHT FROM SWITCH 4n" described in Figs. 3 and 4) to visually indicate that the image scrolling function is in the ON state. Further, on the basis of the control by the light-emission state control unit 4e, the switch 4n emits light in white color (corresponding to "EMIT WHITE LIGHT FROM SWITCH 4n" described in Figs. 3 and 4) to visually indicate that the image scrolling function is in the OFF state.

According to the above-described configuration, every time the user presses down the switch 4n, for example, the light-emission state of the switch 4n is switched to emit one of the green light and the white light in conjunction with the ON-OFF switching of the image scrolling function.

Further, on the basis of the control by the light-emission state control unit 4e, the switch 4n turns off light (corresponding to "TURN OFF LIGHT FROM SWITCH 4n" described in Figs. 3 and 4) to visually indicate that the image scrolling function is unusable (that the image scrolling function is constantly in the OFF state).

Further, on the basis of the control by the light-emission state control unit 4e, the switch 4q included in the switch group 4j emits light in green color (corresponding to "EMIT GREEN LIGHT FROM SWITCH 4q" described in Figs. 3 and 4) to visually indicate that the image rotating function is in the ON state. Further, on the basis of the control by the light-emission state control unit 4e, the switch 4q emits light in white color (corresponding to "EMIT WHITE LIGHT FROM SWITCH 4q" described in Figs. 3 and 4) to visually indicate that the image rotating function is in the OFF state.

According to the above-described configuration, every time the user presses down the switch 4q, for example, the light-emission state of the switch 4q is switched to emit one of the green light and the white light in conjunction with the ON-OFF switching of the image rotating function.

Further, on the basis of the control by the light-emission state control unit 4e, the switch 4q turns off light (corresponding to "TURN OFF LIGHT FROM SWITCH 4q" described in Figs. 3 and 4) to visually indicate that the image rotating function is unusable (that the image rotating function is constantly in the OFF state).

The system controller 4 according to the present embodiment may be configured such that the setting contents set by each user in an observation can be stored in the memory 4c as the setting content for the individual user.

Further, the system controller 4 according to the present embodiment may be configured such that the setting content set by each user in an observation can be stored in the memory 4c as a preset setting content (the setting content available immediately after the start-up of the respective parts of the medical system 1).

Further, the system controller 4 according to the present embodiment may be configured, for example, such that the power supply states of the processors 23 and 32 connected to the system controller 4 are compared by the CPU 4b, and that, on the basis of the result of the comparison, power supply is received from one of the processors having a relatively better power supply state.

As described above, the system controller 4 according to the present embodiment is configured to be able to switch the display state of the LCD panel 4f and the light-emission state of each of the switches included in the switch group 4j in accordance with the respective functions achieved in the ultrasonic endoscope system 2, with the display state and the light-emission state switched in conjunction with the respective functions. According to the above-described configuration, if the system controller 4 according to the present embodiment is used, it is easy for the user to visually determine whether or not a desired function can be used in the ultrasonic endoscope system 2. Therefore, the system controller 4 according to the present embodiment is capable of improving the operability with respect to a medical control device more than before.

Needless to say, the present invention is not limited to the above-described embodiment, and thus can be modified or applied in various ways within a scope not deviating from the gist of the invention.

## Claims

1. A system controller for communicating with a medical control device connectable to plural types of ultrasonic endoscopes, the system controller comprising:
a plurality of operation instruction units capable of issuing an instruction to the medical control device;
a communication unit capable of receiving a control command generated by the medical control device on the basis of table data representing the correspondence among the connection state of the ultrasonic endoscopes, the operational state of the medical control device, and the state of each of the plurality of operation instruction units; and
a control unit for performing a control to visually reflect, in each of the plurality of operation instruction units, the use state of respective functions achievable by the ultrasonic endoscopes and the medical control device, on the basis of the control command.

2. The system controller according to Claim 1, further comprising:
a first switch group including one or a plurality of mechanically configured switches; and
a touch panel,
wherein the plurality of operation instruction units include at least the first switch group and a second switch group including one or a plurality of switches displayed in a screen of the tough panel.

3. The system controller according to Claim 2,
wherein the control unit further performs a control to appropriately change the number of the switches included in the second switch group in accordance with the number of the functions achievable by the ultrasonic endoscopes and the medical control device.

4. The system controller according to Claim 2,
wherein each of the switches included in the first switch group includes a lighting portion capable of emitting light in a plurality of different colors, and
wherein the control unit performs a control to cause the lighting portion corresponding to one of the functions achievable by the ultrasonic endoscopes and the medical control device to emit light in a first color in an ON state of the function, and a control to cause the lighting portion corresponding to the function to emit light in a second color in an OFF state of the function.

5. The system controller according to Claim 3,
wherein each of the switches included in the first switch group includes a lighting portion capable of emitting light in a plurality of different colors, and
wherein the control unit performs a control to cause the lighting portion corresponding to one of the functions achievable by the ultrasonic endoscopes and the medical control device to emit light in a first color in an ON state of the function, and a control to cause the lighting portion corresponding to the function to emit light in a second color in an OFF state of the function.

6. The system controller according to Claim 4,
wherein, in a state in which the function is unusable, the control unit performs a control to cause the lighting portion to turn off the light.

7. The system controller according to Claim 5,
wherein, in a state in which the function is unusable, the control unit performs a control to cause the lighting portion to turn off the light.

8. The system controller according to Claim 1,
wherein the control command received by the communication unit is different between a case in which the medical control device outputs a tomographic image generated in accordance with an echo signal transmitted from the ultrasonic endoscopes as a moving image and a case in which the medical control device outputs the tomographic image as a still image.

9. The system controller according to Claim 2,
wherein the control command received by the communication unit is different between a case in which the medical control device outputs a tomographic image generated in accordance with an echo signal transmitted from the ultrasonic endoscopes as a moving image and a case in which the medical control device outputs the tomographic image as a still image.

10. The system controller according to Claim 3,
wherein the control command received by the communication unit is different between a case in which the medical control device outputs a tomographic image generated in accordance with an echo signal transmitted from the ultrasonic endoscopes as a moving image and a case in which the medical control device outputs the tomographic image as a still image.

11. The system controller according to Claim 4,
wherein the control command received by the communication unit is different between a case in which the medical control device outputs a tomographic image generated in accordance with an echo signal transmitted from the ultrasonic endoscopes as a moving image and a case in which the medical control device outputs the tomographic image as a still image.

12. The system controller according to Claim 5,
wherein the control command received by the communication unit is different between a case in which the medical control device outputs a tomographic image generated in accordance with an echo signal transmitted from the ultrasonic endoscopes as a moving image and a case in which the medical control device outputs the tomographic image as a still image.

13. The system controller according to Claim 6,
wherein the control command received by the communication unit is different between a case in which the medical control device outputs a tomographic image generated in accordance with an echo signal transmitted from the ultrasonic endoscopes as a moving image and a case in which the medical control device outputs the tomographic image as a still image.

14. The system controller according to Claim 7,
wherein the control command received by the communication unit is different between a case in which the medical control device outputs a tomographic image generated in accordance with an echo signal transmitted from the ultrasonic endoscopes as a moving image and a case in which the medical control device outputs the tomographic image as a still image.

15. The system controller according to Claim 1,
wherein the control command received by the communication unit is different between a case in which the medical control device operates in a brightness mode and a case in which the medical control device operates in a color Doppler mode.

16. The system controller according to Claim 2,
wherein the control command received by the communication unit is different between a case in which the medical control device operates in a brightness mode and a case in which the medical control device operates in a color Doppler mode.

17. The system controller according to Claim 3,
wherein the control command received by the communication unit is different between a case in which the medical control device operates in a brightness mode and a case in which the medical control device operates in a color Doppler mode.

18. The system controller according to Claim 4,
wherein the control command received by the communication unit is different between a case in which the medical control device operates in a brightness mode and a case in which the medical control device operates in a color Doppler mode.

19. The system controller according to Claim 5,
wherein the control command received by the communication unit is different between a case in which the medical control device operates in a brightness mode and a case in which the medical control device operates in a color Doppler mode.

20. The system controller according to Claim 6,
wherein the control command received by the communication unit is different between a case in which the medical control device operates in a brightness mode and a case in which the medical control device operates in a color Doppler mode.

21. The system controller according to Claim 7,
wherein the control command received by the communication unit is different between a case in which the medical control device operates in a brightness mode and a case in which the medical control device operates in a color Doppler mode.

22. The system controller according to Claim 14,
wherein the control command received by the communication unit is different between a case in which the medical control device operates in a brightness mode and a case in which the medical control device operates in a color Doppler mode.
